# EUROPÄISCHE PATENTANMELDUNG

(11) **EP 0 579 995 A1**
(43) Veröffentlichungstag der Anmeldung: **26.01.1994**
(21) Anmeldenummer: 93110563.9
(22) Anmeldetag: 02.07.1993
(51) Int. Cl.: A61L 29/00, A61M 25/00

(54) **Katheter-Set**

(30) Priorität: 04.07.1992 DE 4222040
(71) Anmelder: STERIMED Medizinprodukte GmbH, 66337 Püttlingen (DE)
(72) Erfinder: Engelmann, Horst, D-78465 Konstanz (DE); Mehner, Gotthilf, D-66280 Sulzbach (DE)
(74) Vertreter: Rupp, Herbert, Dr.

(57) **Zusammenfassung**

Es wird ein Katheter-Set beschrieben, das eine Splitkanüle 4, einen Katheterschlauch 2, ein Katheteransatzstück 3 und eine Fixationsplatte 6 umfaßt, das dadurch gekennzeichnet ist, daß Katheteransatzstück 3 und Fixationsplatte 6 aus biocid ausgerüstetem Material bestehen.

## Beschreibung

### Technisches Gebiet

Die vorliegende Erfindung betrifft ein Katheter-Set.

### Stand der Technik

In der modernen Medizin ist es notwendig geworden, immer häufiger in immer kritischeren Situationen Katheter einzusetzen. Insbesondere in der Intensivmedizin ergibt sich die Notwendigkeit, Zentralvenenkatheter einzusetzen und über längere Zeiträume im Patienten zu belassen. Trotz sorgfältigster Beachtung aller hygienischen Vorsorgemaßnahmen sind Nebenwirkungen, insbesondere Infektionen, die bis zur lebensbedrohenden Sepsis gehen können, in der Praxis nicht zu vermeiden. Die Bekämpfung dieser Infektionen ist aufwendig und kostenintensiv, ganz abgesehen von der Belastung und Gefahr für den Patienten.

Es hat daher nicht an Versuchen gefehlt, zur Vermeidung von Infektionen Katheter aus biocidem Material herzustellen oder wenigstens deren Oberfläche biocid zu gestalten. So wird z.B. in der EP-A 0301717 vorgeschlagen, pulverförmige Zeolithe, die durch Ionenaustausch freisetzbare biocide Metallionen enthalten, in das Kathetermaterial einzuarbeiten. In WO-A 8401721 werden antimikrobielle Zusammensetzungen enthaltend insbesondere Silber- oder Goldsalze beschrieben, die sich zum Überziehen von Katheter eignen sollen.

In der EP-A 0302186 wird ein Polyurethan-Elastomeres mit in die Polymerkette eingebauten organischen Silikonpolymer-Einheiten ("Silikonpolyurethan") angegeben, das biocid wirkende Metallionen enthält, die bei Berührung mit Körperflüssigkeiten kontinuierlich abgegeben werden.

Mit den biocid ausgerüsteten Kathetern nach dem Stand der Technik wird zwar das angestrebte Ziel, Infektionen zu vermeiden erreicht, jedoch unter Inkaufnahme von schwerwiegenden Nachteilen.

Durch die Zugabe von biocid wirkenden Materialien ändern sich die mechanischen Eigenschaften der thermoplastischen Werkstoffe. So werden beispielsweise Zugfestigkeit, Bruchdehnung und Härte ungünstig beeinflußt. Dadurch werden zum einen die Eigenschaften des Katheters beim Gebrauch beeinträchtigt und zum anderen erhöht sich der Aufwand bei der Herstellung des Katheters.

Als besonders schwerwiegend machen sich die veränderten Oberflächeneigenschaften der biociden Materialien nachteilig bemerkbar. Durch die erhöhte Rauhigkeit verschlechtern sich die Schiebeeigenschaften des Katheters. Außerdem begünstigen diese rauhen Oberflächen Ablagerungen und neigen zum Verwachsen mit Körpergewebe.

### Beschreibung der Erfindung

Die Aufgabe der vorliegenden Erfindung wird in der Zurverfügungstellung von Kathetern gesehen, die einerseits wirksam Infektionen verhindern und andererseits die geschilderten Nachteile der biocid ausgerüsteten Katheter nach dem Stand der Technik vermeiden.

Erfindungsgemäß gelöst wird diese Aufgabe durch ein Katheter-Set umfassend eine Splitkanüle, einen Katheterschlauch, ein Katheteransatzstück und eine Fixationsplatte, dadurch gekennzeichnet, daß Katheteransatzstück und Fixationsplatte aus biocid ausgerüstetem Material bestehen.

Es wurde überraschenderweise festgestellt, daß man bereits durch die biocide Ausstattung des Katheteransatzstücks und der Fixationsplatte die gewünschte Infektionsvermeidung erreicht. Durch diese Maßnahme ist der Katheter in seinen mechanischen Eigenschaften und seiner Anwendungstauglichkeit nicht beeinträchtigt. Durch die Beschränkung der biociden Ausrüstung auf extrakorporale Teile des Katheters wird außerdem die Belastung des Körpers durch biocide Wirkstoffe auf ein Minimum reduziert.

Gegenstand der Erfindung ist daher ein Katheter-Set umfassend eine Splitkanüle, einen Katheterschlauch, ein Katheteransatzstück und eine Fixationsplatte, dadurch gekennzeichnet, daß Katheteransatzstück und Fixationsplatte aus biocid ausgerüstetem Material bestehen.

In einer besonderen Ausführungsform ist die Fixationsplatte zweiteilig gestaltet. Sie besteht in diesem Fall aus einer Grundplatte mit einem Durchbruch, in den ein knopfartiger Einsatz mit einer zentralen Durchtrittsöffnung für den Katheterschlauch eingeschnappt werden kann. Untersuchungen zeigten, daß die gefürchteten Infektionen durch Katheter auch verhindert werden können, wenn nur der knopfartige Einsatz biocid ausgerüstet ist und die Grundplatte aus normalem Material besteht. Durch diese Maßnahme wird der Bedarf an dem teuren biocid ausgerüsteten Material weiter verringert.

Weitere Gegenstände ergeben sich aus den Patentansprüchen.

Das erfindungsgemäße Katheter-Set entspricht in seinem gestalterischen Aufbau völlig dem herkömmlicher Katheter-Sets. Auch die für die nicht biocid ausgerüsteten Teile verwendeten Materialien entsprechen völlig den für Katheter-Sets nach dem Stand der Technik verwendeten Materialien. Die Fixationsplatte und das Katheteransatzstück werden aus den bekannten biocid ausgerüsteten Werkstoffen hergestellt. Beispielsweise werden hierfür die in der EP-A 0302186 vorgeschlagenen "Silikonpolyurethane" in den jeweils angezeigten Shore-Härten herangezogen. Der Aufbau der Fixationsplatte entspricht dem herkömmlicher Fixationsplatten. Die Grundplatte ist z.B. kreisrund, oval, rechteckig oder quadratisch mit einer zentralen Durchtrittsöffnung für den Katheterschlauch. Die Durchtrittsöffnung umfaßt den Katheterschlauch formschlüssig. Die Fixationsplatte ist auf dem Katheterschlauch verschiebbar. Die Durchtrittsöffnung kann als Rohransatz gestaltet sein. Zweckmäßigerweise ist die Achse des Rohransatzes gegenüber der Grundplatte geneigt. Das Legen des Katheters erfolgt auf übliche Weise. Sobald der Katheterschlauch in der gewünschten Lage positioniert ist, wird die Fixationsplatte unmittelbar auf die Oberhaut um die Eintrittsstelle des Katheterschlauches fixiert. Die Fixierung erfolgt wie üblich durch eine Hautnaht oder mit Hilfe von Klebepflaster.

Nachstehend wird die Erfindung anhand der Figuren 1 bis 3 näher erläutert.

Fig. 1 zeigt ein Katheter-Set.

Fig. 2 zeigt einen Querschnitt durch eine Fixationsplatte.

Fig. 3 zeigt eine Draufsicht einer Fixationsplatte.

Fig. 4 zeigt einen Querschnitt durch eine Fixationsplatte.

Fig. 5 zeigt einen Querschnitt durch einen knopfförmigen Einsatz.

Fig. 6 zeigt einen Querschnitt durch eine zweiteilige Fixationsplatte.

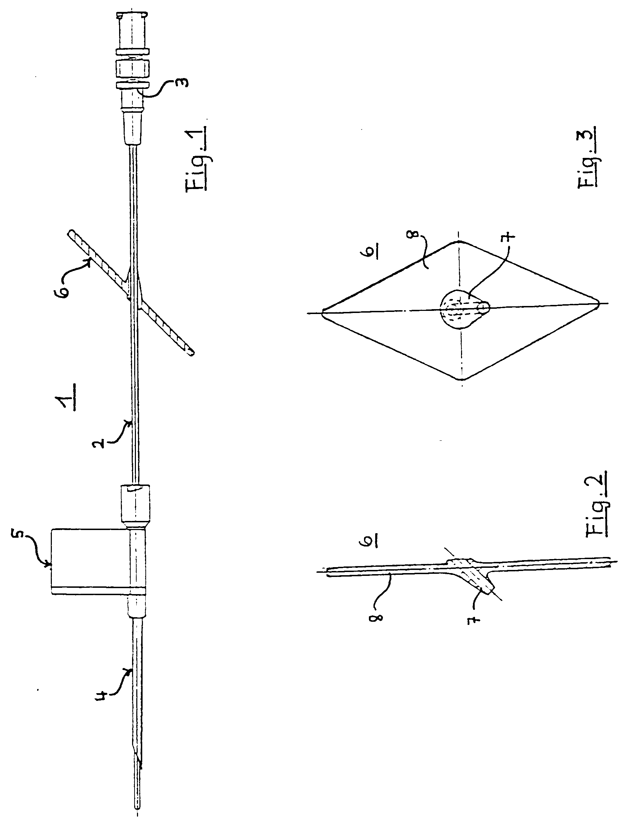

In Fig. 1 ist ein Katheter-Set 1 dargestellt, umfassend einen Katheterschlauch 2, ein Katheteransatzstück 3, eine Splitkanüle 4 mit Griffflügel 5 und Fixationsplatte 6.

In Fig. 2 ist ein Querschnitt durch eine Fixationsplatte 6 dargestellt. Die Achse des Rohransatzes 7 ist gegen die Grundplatte 8 der Fixationsplatte 6 geneigt.

Fig. 3 zeigt eine Draufsicht der Fixationsplatte 6 bestehend aus einer rautenförmigen Grundplatte 8 mit abgerundeten Ecken und einer Durchtrittsöffnung mit Rohransatz 7.

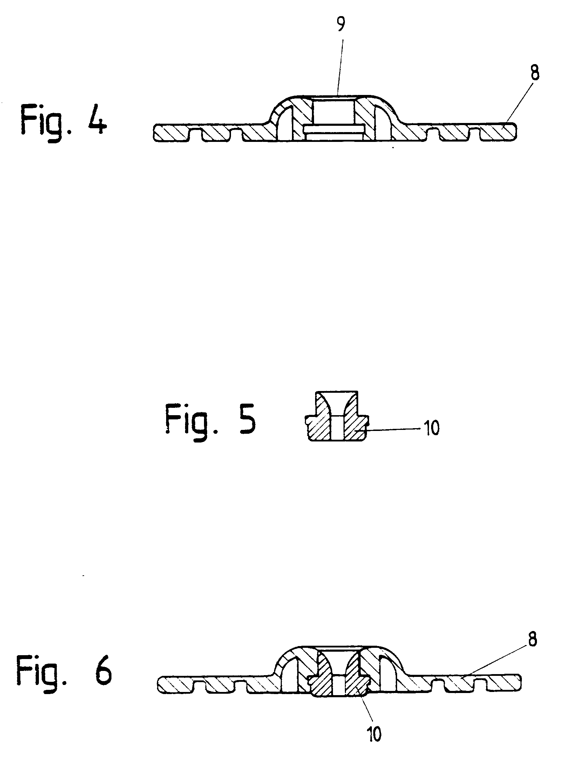

Fig. 4 zeigt einen Querschnitt durch eine Grundplatte 8 einer Fixationsplatte 6, die einen Durchtritt 9 zum Einschnappen des in Fig. 5 dargestellten knopfförmigen Einsatzes 10 aufweist.

In Fig. 6 ist ein Querschnitt durch die Grundplatte 8 nach Fig. 5 mit eingeschnapptem knopfförmigen Einsatz 10 dargestellt.

## Patentansprüche

1. Katheter-Set umfassend eine Splitkanüle (4), einen Katheterschlauch (2), ein Katheteransatzstück (3) und eine Fixationsplatte (6), dadurch gekennzeichnet, daß Katheteransatzstück (3) und Fixationsplatte (6) aus biocid ausgerüstetem Material bestehen.

2. Katheter-Set nach Anspruch 1, dadurch gekennzeichnet, daß die Fixationsplatte (6) aus einer Grundplatte (8) mit einem Durchtritt (9) zum Einschnappen eines knopfförmigen Einsatzes (10) mit Durchtrittsöffnung besteht.

3. Katheter-Set nach Anspruch 2, dadurch gekennzeichnet, daß nur der knopfförmige Einsatz (10) aus biocid ausgerüstetem Material besteht.

4. Katheter-Set nach Anspruch 1, dadurch gekennzeichnet, daß das biocid ausgerüstete Material ein Polyurethan-Elastomeres mit in die Polymerkette eingebauten organischen Silikoneinheiten ist.

5. Katheter-Set nach Anspruch 4, dadurch gekennzeichnet daß das biocid ausgerüstete Material Silberionen abgibt.
